# EUROPEAN PATENT APPLICATION

(11) **EP 1 413 269 A2**
(43) Date of publication of application: **28.04.2004**
(21) Application number: 03023915.6
(22) Date of filing: 21.10.2003
(51) Int. Cl.: A61F 6/04

(54) **Condom with wearing auxiliary device**

(30) Priority: 22.10.2002 JP 2002306657; 22.10.2002 JP 2002306658
(71) Applicant: Shoei Limited Co., Tokyo (JP)
(72) Inventor: Tsugawa, Wataru, Edogawa-ku, Tokyo (JP)
(74) Representative: Grünecker, Kinkeldey, Stockmair & Schwanhäusser Anwaltssozietät

(57) **Abstract**

The present invention provides a condom with a wearing auxiliary device including a condom made of a natural rubber or a synthetic rubber and a wearing auxiliary device, including a structure in which the wearing auxiliary device is used as a core material of the rolled-up part which is formed by rolling-up a condom body from the open end of the condom toward the closed end having a teat so that the outer surface of the condom turns inside, wherein the wearing auxiliary device includes a wearing auxiliary device selected from the group consisting of a wearing auxiliary device (a) including a coil-shaped member composed of a material selected from the group consisting of a rubber-like elastic substance, a polymer foam, a polymeric substance and a metal; and a wearing auxiliary device (b) composed of a polymeric material having rubber elasticity.

## Description

### BACKGROUND OF THE INVENTION

### Field of the Invention

The present invention relates to a condom with a wearing auxiliary device comprising a condom and a wearing auxiliary device that is easily applied. More particularly, the present invention relates to a condom with a wearing auxiliary device that is easily applied, in which the body of the condom is rolled up utilizing the wearing auxiliary device as a core material.

### Related Background Art

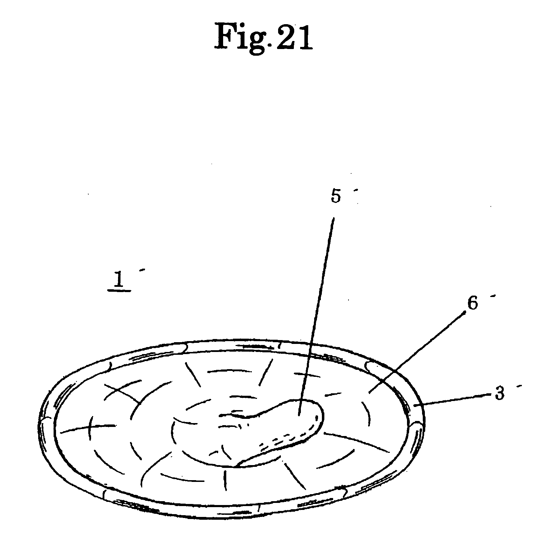

A common condom is packaged in a form shown in Fig. 21, in which it is rolled up from the open end of the condom body toward the closed end having a teat, that is, from the open end toward the tip end so that the outer wall of the condom turns inside, to form a rolled-up part. For application, the inner wall of the condom at the tip end is brought into contact with the tip of the penis, and then the condom is applied to the penis while the rolled-up part is unrolled.

However, since there is a trend that the thinner condom is the more preferred, the rolled-up part based on the ring has a thickness (diameter) of only approximately 4 mm, which is very small compared to the size of a finger. Thus, when the condom is to be applied to the penis, there occurs a problem that the application is very difficult, or the handling is difficult.

Therefore, various devices have been proposed for eliminating this inconvenience.

For example, Japanese Patent Application Laid-Open No. 10-165433 discloses an auxiliary ring for applying a condom to be used at the application. This auxiliary ring consists of a rubber ring provided with irregularities for identifying the front side of a condom, four guide rods having projections for preventing falling off and a bottom face sheet provided with an inserting opening at the center. The auxiliary ring has a structure in which the guide rods are assembled inside the rubber ring in a form to provide a space in the center for inserting the penis; the bottom face sheet is attached to the bottom of the rubber ring; and the condom is sandwiched between the guide rods and the bottom face sheet from above and below. Although this structure is relatively simple, there will be problems, for example, the bottom face sheet may be broken if the rubber ring is prone to deform; the above tool must be specially prepared for application; or the tool is easily contaminated with jelly coated on the condom, resulting in difficulty in keeping cleanness all the time.

In addition, Japanese Patent Application Laid-Open No. 7-308336 discloses a condom application tool. This application tool comprises a U-shaped cylinder which is provided with a base to support it, and a groove is provided to fix a condom. Further, Japanese Patent Application Laid-Open No. 6-14952 and Japanese Patent Application Laid-Open No. 6-154260 disclose a ring-form condom applicator and the like for holding a condom and enabling easy application. However, the condom must be set temporarily on the application tool or the applicator before it is applied to the penis, and they must be removed after the application. Consequently, these application tools and applicators have the drawback that they must be cleaned after the use because they are contaminated with a coated jelly during and after the application of the condom; they by themselves are required to be clean all the time; the condom must be specially applied to the applicator for the application and use; or the applicator must be prepared all the time.

Furthermore, Japanese Patent Application Laid-Open No. 8-56978, Japanese Patent Application Laid-Open No. 8-252277, Japanese Patent Application Laid-Open No. 6-197921, and Japanese Patent Application Laid-Open No. 9-24059 disclose a method for producing a condom in which a tape is rolled in the rolled-up part for making the application of the condom to the penis easier. The method comprises producing a condom with a tape by rolling up the condom with at least a piece of tape-shaped material or a pair of the tape-shaped materials accompanied when the condom is rolled-up from the open end thereof toward the closed end having a teat to form the rolled-up part. This condom with a tape can be easily applied by unrolling the rolled-up part by pulling the tape to the root of the penis at the application.

This method has the advantage of eliminating an applicator or other auxiliary devices. However, although the tape having smaller width and thickness is preferred, there occur several problems, for example, the difficulty in pulling the tape at the application due to the smaller width and thickness, the difficulty in rolling-up for forming a rolled-up part, or the difficulty in grasping the tape due to sticking thereof to jelly when the jelly is coated on the condom. On the other hand, when the width and/or the thickness of the tape is increased, for example only the thickness is increased, there occurs the drawback that only a part of the rolled-up part becomes bulky and looks bad. Further, although there is the advantage of easy pulling at the application when the both are increased, there occurs the problem that the shape of the rolled-up part is not circular but flat, which looks extremely bad, and in addition, the condom becomes difficult to be applied.

Furthermore, Japanese Patent Application Laid-Open No. 9-47466 discloses a condom that is formed of a flexible film such as polyvinyl chloride film. In this patent, since it is difficult to directly form a condom with a polyvinyl chloride film, two sheets of film are seamed by heat-sealing to form a side part of the condom having a shape that is slightly loose compared to the penis in erection. However, as some burrs are produced at the heat-sealed part, the condom must be formed so that the burrs faces the penis side. In addition, this condom is easily fallen off due to the size that is a little loose. To prevent the falling off, a circular ring of a silicone rubber need to be put on the film condom at the neighborhood of the root of the penis. However, as the condom is made by seaming two sheets of film by heat-sealing, pin holes are easily formed at the seam part, so that there may be a problem in the effect for contraception or preventing AIDS infection. There is also the problem that a circular ring must be put on the penis. Moreover, as burrs are formed at the seam part, the formed condom must be turned inside out to put the burrs to the inside of the condom, which may injure the penis. Also, some wrinkle may be formed although the film is flexible, and the wrinkle at the tip may be hard enough to injure the vaginal wall or to cause pain. Thus, the condom using the flexible vinyl chloride film is not practical.

### SUMMARY OF THE INVENTION

As described above, as a condom is small compared to the size of the human hand, conventional one is difficult to be applied to the penis. Therefore, various devices have been tried to make the application of the condom easier. Thus, the condom, in which the production of a product is easy; the appearance of the product is compatible with the atmosphere for the application; the application of the condom to the penis is easy; and the wearing auxiliary device can be easily removed after the application of the condom, is desired.

The present invention provides a condom that is easy to handle by using a wearing auxiliary device in order to solve the above described problems. Consequently, the condom is easily applied to the penis and the wearing auxiliary device can be easily removed after the condom is applied. When the condom is rolled up to form a product, the diameter of the rolled-up part is increased by using the wearing auxiliary device as a core material. This large diameter of the rolled-up part makes the application of the condom to the penis easy. Further, after the condom is applied, the used wearing auxiliary device can be very conveniently removed. The present invention has aimed at these advantages, and relates to a condom with a wearing auxiliary device comprising a condom and a wearing auxiliary device.

The present invention relates to a condom with a wearing auxiliary device comprising a condom made of a natural rubber or a synthetic rubber and a wearing auxiliary device, comprising a structure in which the wearing auxiliary device is used as a core material of the rolled-up part which is formed by rolling-up the condom body from the open end of the condom toward the closed end having a teat so that the outer surface of the condom turns inside, wherein the wearing auxiliary device comprises a wearing auxiliary device selected from the group consisting of a wearing auxiliary device (a) comprising a coil-shaped member composed of a material selected from the group consisting of a rubber-like elastic substance, a polymer foam, a polymeric substance and a metal; and a wearing auxiliary device (b) composed of a polymeric material having rubber elasticity.

The wearing auxiliary device (a) comprises a coil-shaped member composed of a material selected from the group consisting of a rubber-like elastic substance, a polymer foam, a polymeric substance and a metal. This coil-shaped member is made by winding, round and round in a circular or helical fashion, a thread made of a material selected from the group consisting of a rubber-like elastic substance, a polymer foam, a polymeric substance and a metal. The profile of the thread is generally circular, flat rectangular or of any other shapes, preferably generally circular or flat rectangular.

In addition, this coil-shaped member has a structure in which both ends thereof are joined to form a ring or they are left as free ends without being joined. For forming a rolled-up part, a generally preferred coil-shaped member includes a ring shape in which both ends are joined, or the one that is formed into a ring shape from the beginning. However, even the coil-shaped member whose both ends form free ends can easily form a rolled-up part by making a C-shape or by forming a ring by inserting a flexible member into the space between the both ends of the coil-shaped member.

Further, the diameter of the coil can be set at any given value depending on the purpose, but is naturally limited by the relation with the thickness of the material of the condom. When the diameter is too small, the effect of using the coil is not clear when the rolled-up part is formed. Therefore, it is desirable that the diameter is generally from approximately 0.5 to 5 mm, preferably from approximately 2 to 4 mm.

As the wearing auxiliary device (a) is constructed of a coil-shaped member, it has the characteristic of easy expansion and contraction and is easily removed from the penis.

The polymeric substances include thermoplastic polymers such as polyolefin such as polyethylene, polypropylene and polybutylene, and polystyrene; fiber-forming polymeric substances such as polyester and nylon; those substances that easily form coil shape such as a conjugate fiber of polyester and nylon; and the like.

Further, any metal can be used such as iron, iron alloys such as stainless steel, aluminum, nickel, nickel alloys and silver. These metals may be the metal itself or may be those having a surface on which a coating film is formed using a coating such as vanish or lacquer. The coating may be non-colored or colored, and when colored, the combination with the color of the condom is preferably considered.

The wearing auxiliary device (a) may be non-colored. However, it may be necessary to devise a color that can easily distinguish the wearing auxiliary device, preferably considering the combination with the color of the condom. It is preferable to color it as appropriate considering the color that is reasonably compatible with the color of the condom.

The wearing auxiliary device (b) is composed of a polymeric material having rubber elasticity. This polymeric material having rubber elasticity is a material selected from the group consisting of polyurethane, a polyurethane foam, a natural rubber, a synthetic rubber and a synthetic rubber foam.

Any known polyurethane that has rubber elasticity can be used. In addition, the polyurethane foam can be selected as appropriate from the known polyurethane foams having rubber elasticity for use.

The synthetic rubber or the synthetic rubber foam can be selected as appropriate from the known synthetic rubber materials for use.

The synthetic rubbers include various known materials having rubber elasticity such as butadiene synthetic rubber, chloroprene synthetic rubber, isoprene synthetic rubber, ethylene/propylene rubber, acrylic rubber, ethylene-acrylic rubber and silicone rubber.

The wearing auxiliary device (b) has a structure in which its both ends are joined to form a ring or both ends form free ends. Preferably, the one that has a ring shape by joining both ends or the one that is formed into a ring shape from the beginning is desired. The one that is formed into a ring shape from the beginning has the advantage of easily making a rolled-up part when it is formed. In addition, the shape of the profile can be circular, oval or elliptic, square, rectangular, or the other shape, and it is desirable that the wearing auxiliary device (b) has a generally circular profile. Further, the wearing auxiliary device (b) may have a structure in which a constriction or a notch is provided at any given location in the longitudinal direction thereof. Also, the polymeric material having rubber elasticity may be a string-like material on which beads are strung such as a necklace.

The maximum diameter of the profile can be set at any given value depending on the purpose, but is naturally limited by the relation with the thickness of the material of the condom. When the diameter is too small, the effect of using the tool is not clear when the rolled-up part is formed. Therefore, it is desirable that the diameter is generally from approximately 0.5 to 5 mm, preferably from approximately 2 to 4 mm.

When the wearing auxiliary device (b) is a ring-shaped product, the profile is preferably generally circular, but small deformation is acceptable. However, it is preferably not deformed too much because when a condom body is rolled up from the open end of the condom toward the tip thereof using the ring-shaped product as a core material, the operation for the rolling-up may be not smooth if it is deformed too much.

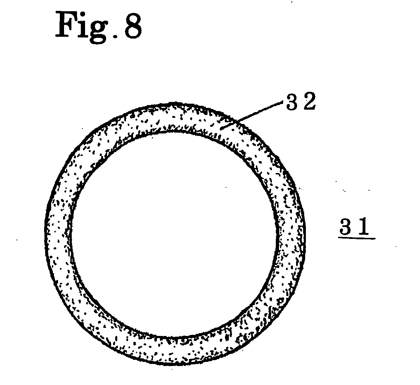

The "constriction", in the case where a ring-shaped product made of a polymeric material having rubber elasticity that is formed by joining both ends has a generally circular profile or a ring-shaped product that is formed into a ring shape from the beginning has a generally circular profile, includes a constriction formed from the surface toward the center of the circular cross-section; a constriction formed from the outer periphery of the ring toward the inner periphery thereof; a constriction formed from the inner periphery of the ring toward the outer periphery thereof; and deformed constrictions such as a constriction that is gently formed from the surface toward the center of the cross-section, which is then gently eliminated (the shape in which a number of generally gourd-shaped products are coupled in the entire shape of the ring-shaped product as shown in Fig. 8), and a constriction that forms the surface of a hemisphere toward the center of the circular cross-section, which is then eliminated forming the surface of a hemisphere.

Constrictions, when they are suitably formed, make the ring-shaped product very flexible one that is rich in elasticity in cooperation with the elasticity of a material.

The "notch" may include a notch formed helically along the surface of a polymeric material having rubber elasticity in the longitudinal direction, and a notch formed from the surface toward the center at any given locations. The notch may be provided in plural or may be single.

Helical notches form a kind of coil-shaped product that is very flexible and rich in elasticity in cooperation with the elasticity of a material.

The "notch", similar to "constriction", in the case where a ring-shaped product made of a polymeric material having rubber elasticity that is formed by joining both ends has a generally circular profile or a ring-shaped product that is formed into a ring shape from the beginning has a generally circular profile, includes a notch formed from the surface toward the center of the circular cross-section; a notch formed from the outer periphery of the ring toward the inner periphery thereof; and a notch formed from the inner periphery of the ring toward the outer periphery thereof. Helical notches in the longitudinal direction of a ring-shaped product make a kind of coil-shaped product that is very flexible and rich in elasticity in cooperation with the elasticity of a material.

When the wearing auxiliary device (a) and the wearing auxiliary device (b) are each ring-shaped products, the entire shape is preferably generally circular, but may be deformed, such as elliptical. However, it is preferably not deformed too much because when a condom body is rolled up from the open end of the condom toward the tip thereof using the ring-shaped product as a core, the operation for the rolling-up may be not smooth if it is deformed too much.

When the wearing auxiliary device (b) is provided with constrictions or notches, it may be cut from the constrictions or the notches in a rolled-up part. However, even if the wearing auxiliary device (b) is cut in the rolled-up part, the application of the condom to the penis is not influenced, and the removal of the wearing auxiliary device (b) from the condom is also not influenced.

Further, when the wearing auxiliary device (b) is an extremely flexible foam, the foam may be compressed and shrunk by the rubber of the condom associated with the roll-up of the condom. In such a case, those having a larger diameter need to be used, or the use of a little harder foam may be preferable.

The condom according to the present invention can use various conventionally well known materials. However, the condom that is formed by using a natural rubber or a synthetic rubber is the most preferable.

The condom may be colored using a colorant, or may be formed by adding any other appropriate additives.

Further, the condom that is coated with jelly may be used.

The rolled-up part results in a structure in which the condom body is rolled around a core using the wearing auxiliary device as the core, so that the diameter or thickness of the rolled-up part is increased by the size of the core. Thus, the diameter or thickness of the rolled-up part of conventionally approximately 4 mm is considerably increased, making the application easier.

Although the wearing auxiliary device must be removed after the application of the condom, the wearing auxiliary device is stretched easily to a considerably larger ring than the outer periphery of the penis, so that it is easily taken off.

Further, different from the condom using a tape-shaped product, the condom using the wearing auxiliary device of the present invention looks beautiful and natural, and provides the advantage of easy application and the like, since the rolled-up part is circular-shaped or generally circular-shaped. It is said that the application of the condom before performing sex acts is not common, and the condom is commonly applied during the sex acts. In such a case, the surface of the penis is lubricated by the secretion from the vagina or the like, and so the application is very difficult. The condom of the present invention provides the advantage of easy application even in such a case. Moreover, it provides the advantage also in the production that the structure is very simple; its production is easy; and the conventional machines for producing condoms can be applied without any modification.

### BRIEF DESCRIPTION OF THE DRAWINGS

Fig. 1 shows a perspective view of a condom with a wearing auxiliary device of the present invention in which a part of a rolled-up part is cut away;
Fig. 2 shows a partial sectional view of the rolled-up part of the condom with a wearing auxiliary device of the present invention shown in Fig. 1;
Fig. 3 shows a front view of the wearing auxiliary device 21 in a first embodiment of the wearing auxiliary device (a);
Fig. 4 shows a side view of the wearing auxiliary device 21 in a first embodiment of the wearing auxiliary device (a);
Fig. 5 shows a profile of the wearing auxiliary device 21 in a first embodiment of the wearing auxiliary device (a);
Fig. 6 shows a partial view of a second embodiment of the wearing auxiliary device (a);
Fig. 7 shows a profile of a second embodiment of the wearing auxiliary device shown in Fig. 6;
Fig. 8 shows a front view of a first embodiment of the wearing auxiliary device (b);
Fig. 9 shows a perspective view of a second embodiment of the wearing auxiliary device (b);
Fig. 10 shows a front view of a second embodiment of the wearing auxiliary device (b);
Fig. 11 shows a front view of a third embodiment of the wearing auxiliary device (b);
Fig. 12 shows a front view of a fourth embodiment of the wearing auxiliary device (b);
Fig. 13 shows a front view of a fifth embodiment of the wearing auxiliary device (b);
Fig. 14 shows a front view of a sixth embodiment of the wearing auxiliary device (b);
Fig. 15 shows a perspective view of a seventh embodiment of the wearing auxiliary device (b);
Fig. 16 shows a partially enlarged perspective view of a eighth embodiment of the wearing auxiliary device (b);
Fig. 17 shows a partially enlarged perspective view of a ninth embodiment of the wearing auxiliary device (b);
Fig. 18 shows a partially enlarged perspective view of a tenth embodiment of the wearing auxiliary device (b);
Fig. 19 shows a partially enlarged perspective view of an 11th embodiment of the wearing auxiliary device (b);
Figs. 20A, 20B and 20C show a process of the method for producing the condom with a wearing auxiliary device; and
Fig. 21 shows a perspective view of the condom in which a conventional rolled-up part is formed.

### DESCRIPTION OF THE PREFERRED EMBODIMENTS

The present invention will now be described in detail with reference to the drawings.

Fig. 21 shows a conventional condom 1', wherein the open end typically has a ring (not shown) formed integrally with a condom body; and the condom is rolled up to the closed end 6' using the ring as a base so that the outer periphery of the condom turns inside to form the rolled-up part 3'. The thus prepared condom 1' is each packaged to form a product.

In this case, the diameter or thickness of the ring is a little larger than the film thickness of the condom. This ring is used as a core to roll up to make a condom having a shape shown in Fig. 21. The diameter of this ring is a little larger than the film thickness of the condom. The condom having a thinner film thickness is preferred because the condom having a thicker film thickness reduces sexual feeling. Consequently, the diameter of the ring has no other choice but to be small. Thus, the size (diameter) of the rolled-up part 3' is small compared to the size of the human fingers, which causes practical problems such as requirement of much time to apply the condom to the penis while unrolling it, or the like.

Note that reference numeral 5' denotes a teat in Fig. 21.

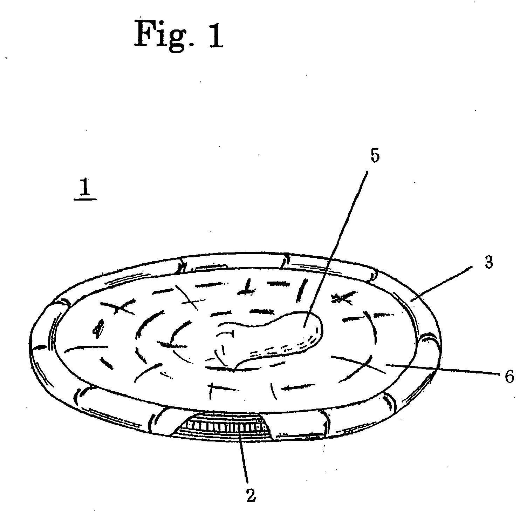

In the present invention, the condom 1 with a wearing auxiliary device shown in Fig. 1 is devised such that the wearing auxiliary device 2 and the ring formed at the open end of the condom are used as a core to control the size (diameter) of the rolled-up part 3 to a size that is easy in application and handling regardless of the film thickness of the condom. Fig. 1 shows a perspective view of the condom in which a part of the rolled-up part 3 is cut away, wherein reference numeral 6 denotes a closed end and reference numeral 5 denotes a teat. The thus produced condom is separately packaged to form a commercial product.

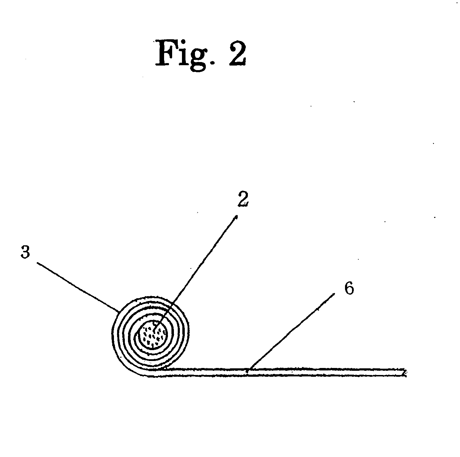

Fig. 2 shows a partial sectional view of the rolled-up part 3 of the condom shown in Fig. 1, wherein reference numeral 2 denotes a wearing auxiliary device and reference numeral 6 denotes a closed end. The rolled-up part 3 has a large size (diameter) so that the condom is easy to apply, because the condom is rolled up using the wearing auxiliary device 2 as a core material.

The wearing auxiliary device (a) or the wearing auxiliary device (b) is used for this wearing auxiliary device 2.

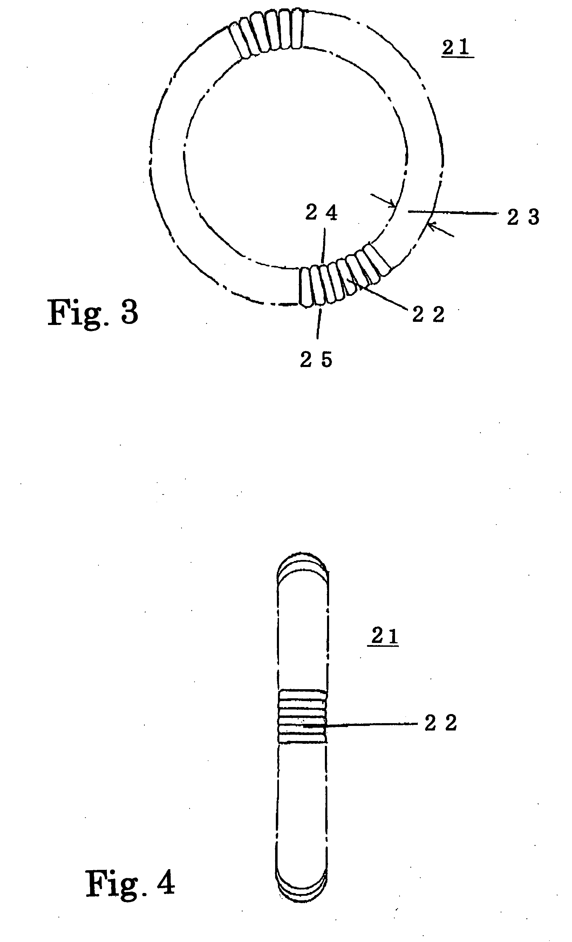

The wearing auxiliary devices of the present invention are shown in Figs. 3 to 19.

Figs. 3 to 5 show a first embodiment of the wearing auxiliary device (a) of the present invention.

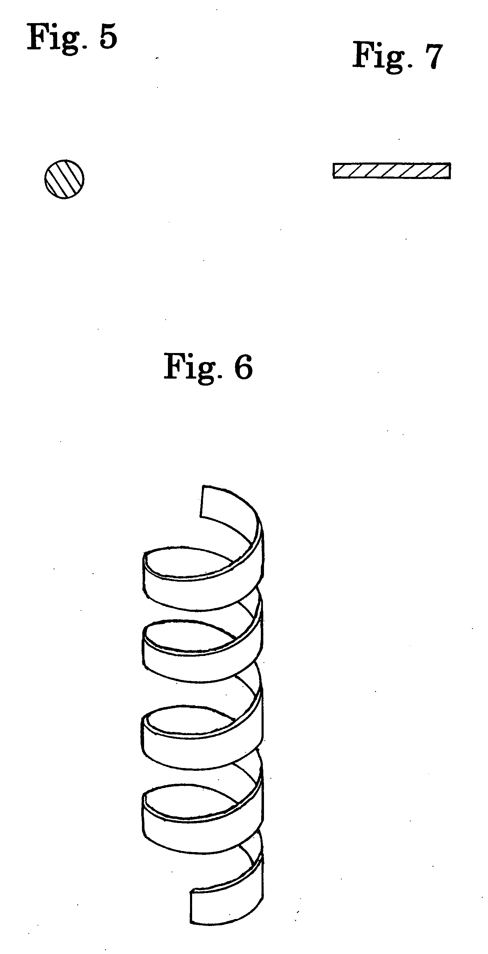

Fig. 3 shows a front view of the wearing auxiliary device 21; Fig. 4 shows a side view of the wearing auxiliary device 21; and Fig. 5 shows a profile of the coil member. The wearing auxiliary device 21 is a ring-shaped product formed by joining both ends of the coil-shaped member, wherein the inner periphery 24 of the wearing auxiliary device has approximately the same size of the outer periphery of the condom; and the coil-shaped member 22 is composed of a material selected from the group consisting of a rubber-like elastic substance, a polymer foam, a polymeric substance and a metal. In addition, the coil member has a circular profile as shown in Fig. 5. As this first embodiment of the wearing auxiliary device (a) has the structure as described above, it can be handled elastically. When the condom of the present invention is applied, the application is easy because the wearing auxiliary device (a) is inserted as a core of the rolled-up part 3 shown in Fig. 1 so that the size of the rolled-up part 3 is increased (diameter is increased). In addition, as the wearing auxiliary device is formed into a ring-shape using a coil-shaped member, the pressure to compress the penis by the wearing auxiliary device in the rolled-up part 3 will not be produced. On the other hand, since the coil member is elastic, the wearing auxiliary device can be easily removed from the penis after the condom is applied.

Incidentally, reference numeral 23 denotes the diameter of the coil shape; reference numeral 24 denotes the inner periphery of the wearing auxiliary device; and reference numeral 25 denotes the outer periphery of the wearing auxiliary device.

Figs. 6 and 7 each show a second embodiment of the wearing auxiliary device (a). Fig. 6 shows a partial view of the second embodiment of the wearing auxiliary device (a), and Fig. 7 shows a profile thereof. This wearing auxiliary device (a) is composed of a coil-shaped member having a profile of generally flat rectangle as shown in Fig. 7. As this second embodiment of the wearing auxiliary device (a) has the structure as described above, it can be handled elastically similar to the first wearing auxiliary device (a). Moreover, the condom using this wearing auxiliary device, similar to the case where the first wearing auxiliary device (a) is used, provides the effect that the application to the penis is easy and its removal is also easy.

Figs. 8 to 19 show several embodiments of the wearing auxiliary device (b).

Fig. 8 shows a first embodiment of the wearing auxiliary device (b). Fig. 8 shows a front view thereof. The wearing auxiliary device 31 comprises a ring 32 that is formed using a polymeric material having rubber elasticity.

The polymeric material having rubber elasticity is selected from the group consisting of polyurethane, a polyurethane foam, a natural rubber, a synthetic rubber and a synthetic rubber foam. The ring 32 is formed by joining the both ends of the polymeric material having rubber elasticity or formed as a ring from the beginning.

Also, the polymeric material having rubber elasticity may be a string-like material on which beads are strung such as a necklace.

As this first embodiment of the wearing auxiliary device (b) has the structure as described above, it can be handled elastically. When the condom of the present invention is applied, the application of the condom is easy because the wearing auxiliary device (b) is inserted as a core of the rolled-up part 3 shown in Fig. 1 so that the size of the rolled-up part 3 is increased (diameter is increased). In addition, as the wearing auxiliary device is formed into a ring-shape using a polymeric material having rubber elasticity, the pressure to compress the penis by the wearing auxiliary device in the rolled-up part 3 will not be produced. On the other hand, since the polymeric material having rubber elasticity is elastic, the wearing auxiliary device can be easily removed from the penis after the condom is applied.

The shape of the profile of this embodiment can be circular, oval or elliptic, square, rectangular, or the other shape, preferably circular.

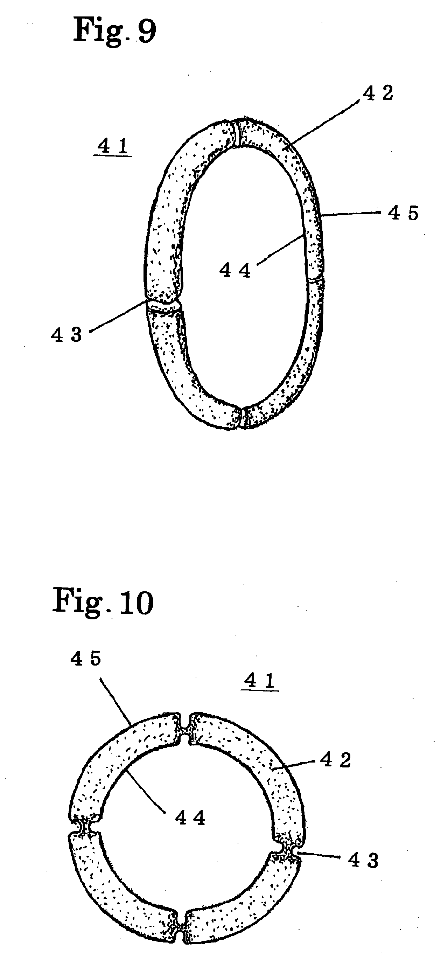

Figs. 9 and 10 show a second embodiment of the wearing auxiliary device (b). Fig. 9 shows a perspective view of a wearing auxiliary device 41; and Fig. 10 shows a front view of the wearing auxiliary device 41. The wearing auxiliary device 41 comprises a ring-shaped product 42 provided with a constriction 43. The ring-shaped product 42 has a generally circular cross-section and the constriction 43 is formed toward the center of the cross-section. The inner periphery 44 has a size that is approximately the same as the outer periphery of the condom. This is intended to make the roll-up to be easily performed when the condom is rolled up from the open end to the tip. If it is larger than the outer periphery of the condom, there will be the inconvenience that the ring-shaped product may be bent at the beginning of the roll-up. On the other hand, if it is smaller than the outer periphery of the condom, the ring-shaped product may soon be cut at the constriction part because a force to expand the size of the ring of the ring-shaped product will soon act on the ring-shaped product, as the condom is rolled up. Reference numeral 45 denotes the outer periphery of the ring-shaped product 42. In this embodiment, the wearing auxiliary device 41 has constrictions and so it expands and contracts by a small force, making the removal of the condom after the application easy. Further, as it also provides the advantage that it can be easily cut during the removal operation, the condom in wear may be removed extremely easily.

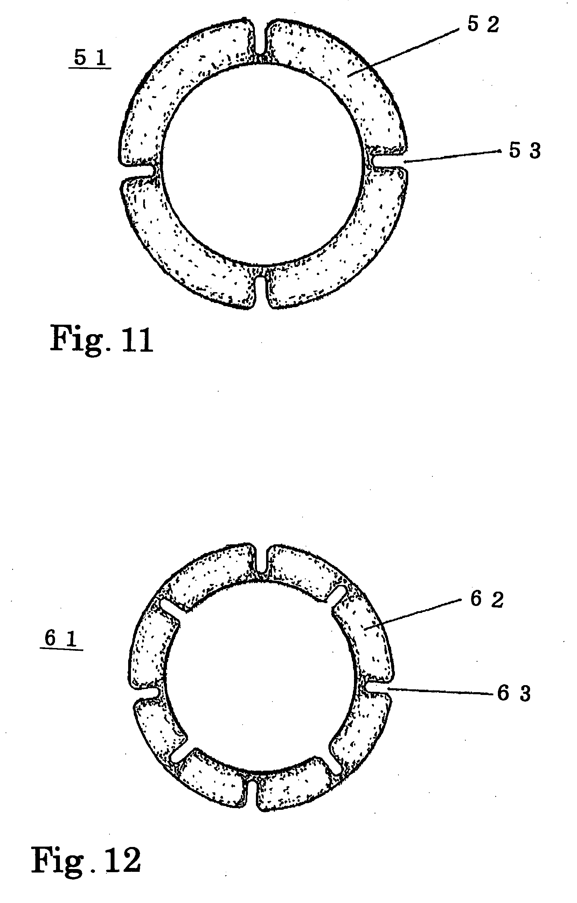

Fig. 11 shows a third embodiment of the wearing auxiliary device (b), wherein a front view of the wearing auxiliary device 51 is shown. In this embodiment, a plurality of constrictions 53 are provided from the outer periphery toward the inner periphery of the ring-shaped product 52. The third embodiment of the wearing auxiliary device (b) has the effect similar to the above described second embodiment.

Fig. 12 shows a fourth embodiment of the wearing auxiliary device (b), wherein a front view of the wearing auxiliary device 61 is shown. In this embodiment, the constrictions 63 consist of the constriction from the outer periphery toward the inner periphery of the ring-shaped product 62 and the constriction from the inner periphery toward the outer periphery thereof, and each of these two types is alternately provided in plural to form the constrictions 63 of this wearing auxiliary device 61. As these two types of constrictions are alternately provided, it expands and contracts by a smaller force than a conventional ring-shaped product, and when it is expanded, the length is further longer than the conventional one. Further, the fourth embodiment of the wearing auxiliary device (b) has the effect similar to the above described second embodiment.

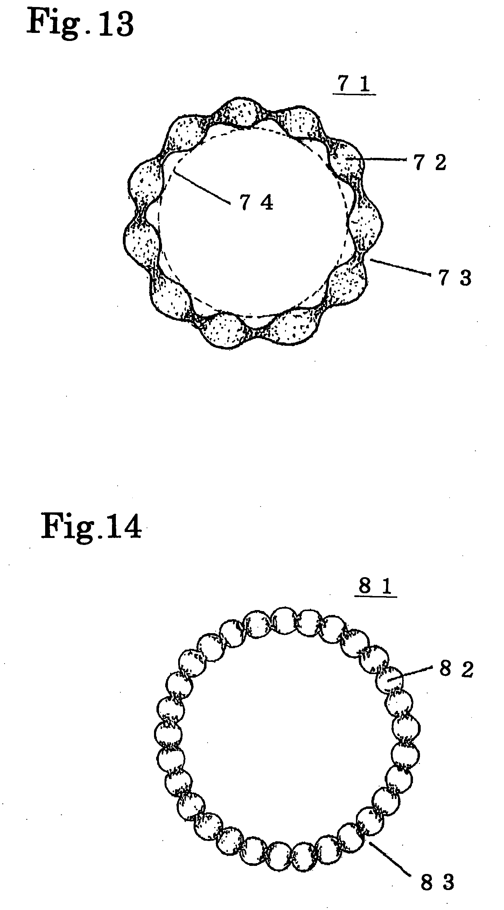

Fig. 13 shows a fifth embodiment of the wearing auxiliary device (b), wherein a front view of the wearing auxiliary device 71 is shown. In this embodiment, gentle constrictions 73 are formed successively in the longitudinal direction of the ring-shaped product 72. In other words, the ring-shaped product has a structure in which a number of generally gourd-shaped products are coupled. Reference numeral 74 denotes the inner periphery of the ring-shaped product 72. The fifth embodiment of the wearing auxiliary device (b) has the effect similar to the above described second embodiment.

Fig. 14 shows a sixth embodiment of the wearing auxiliary device (b), wherein a front view of the wearing auxiliary device 81 is shown. In this embodiment, constrictions 83 are formed in the shape of a hemisphere on the ring-shaped product 82, showing an entire shape in a state as if a number of beads are coupled. The sixth embodiment of the wearing auxiliary device (b) has the effect similar to the above described second embodiment.

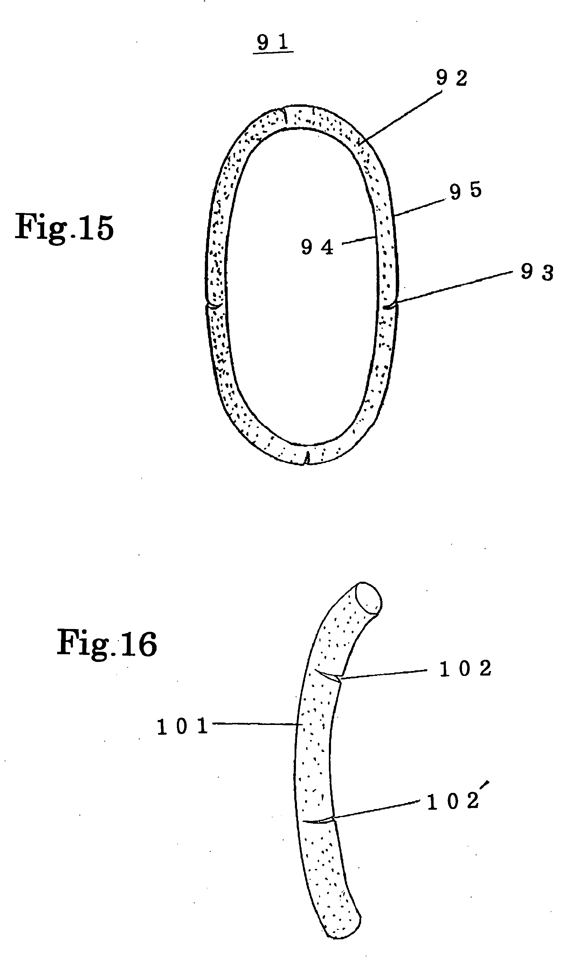

Fig. 15 shows a perspective view of a seventh embodiment of the wearing auxiliary device (b). This wearing auxiliary device (b) 91 comprises the ring-shaped product 92 provided with a plurality of notches 93 from the outer periphery toward the inner periphery thereof. Reference numeral 94 denotes the inner periphery and reference numeral 95 denotes the outer periphery. The seventh embodiment of the wearing auxiliary device (b) has the effect similar to the above described second embodiment.

Fig. 16 shows a partially enlarged perspective view of an eighth embodiment of the wearing auxiliary device (b). This wearing auxiliary device (b) comprises the polymeric material having rubber elasticity 101 provided with a plurality of notches 102 and 102'. The eighth embodiment of the wearing auxiliary device (b) has the effect similar to the above described second embodiment.

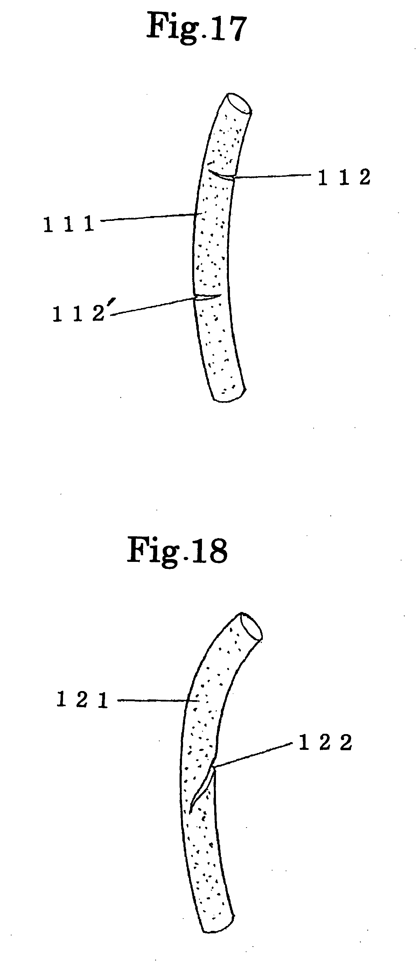

Fig. 17 shows a partially enlarged perspective view of a ninth embodiment of the wearing auxiliary device (b). This wearing auxiliary device (b) comprises the polymeric material having rubber elasticity 111 provided with notches 112 and 112' alternately, the direction of the notches 112 and 112' being opposite. The ninth embodiment of the wearing auxiliary device (b) has the effect similar to the above described fourth embodiment.

Fig. 18 shows a partially enlarged perspective view of a tenth embodiment of the wearing auxiliary device (b). This wearing auxiliary device (b) comprises the polymeric material having rubber elasticity 121 provided with a notch 122 from an oblique direction. The tenth embodiment of the wearing auxiliary device (b) has the effect similar to the above described second embodiment.

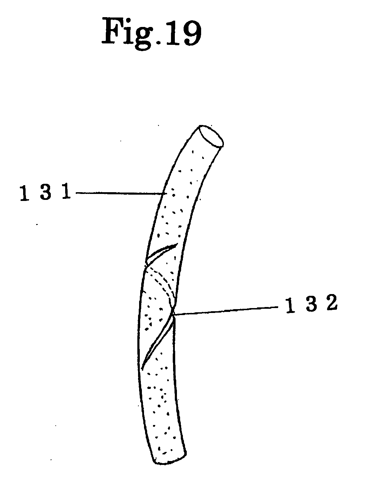

Fig. 19 shows a partially enlarged perspective view of an 11th embodiment of the wearing auxiliary device (b). This wearing auxiliary device (b) comprises the polymeric material having rubber elasticity 131 provided with a helical notch 132. This wearing auxiliary device provided with a helical notch is most preferred, because when it is provided with a plurality of helical notches, or the helical notches are provided over the wearing auxiliary device (b), it provides the same effect as the wearing auxiliary device (a) constructed of a coil-shaped member.

The constrictions to be provided in the wearing auxiliary devices (b) of the present invention shown in each of Figs. 9 to 14 may be used singly or in combination. However, the constrictions in the present invention are not limited to those shown in these figures.

The notches to be provided in the wearing auxiliary devices (b) of the present invention shown in each of Figs. 15 to 19 may be used singly or in combination. However, the notches in the present invention are not limited to those shown in these figures.

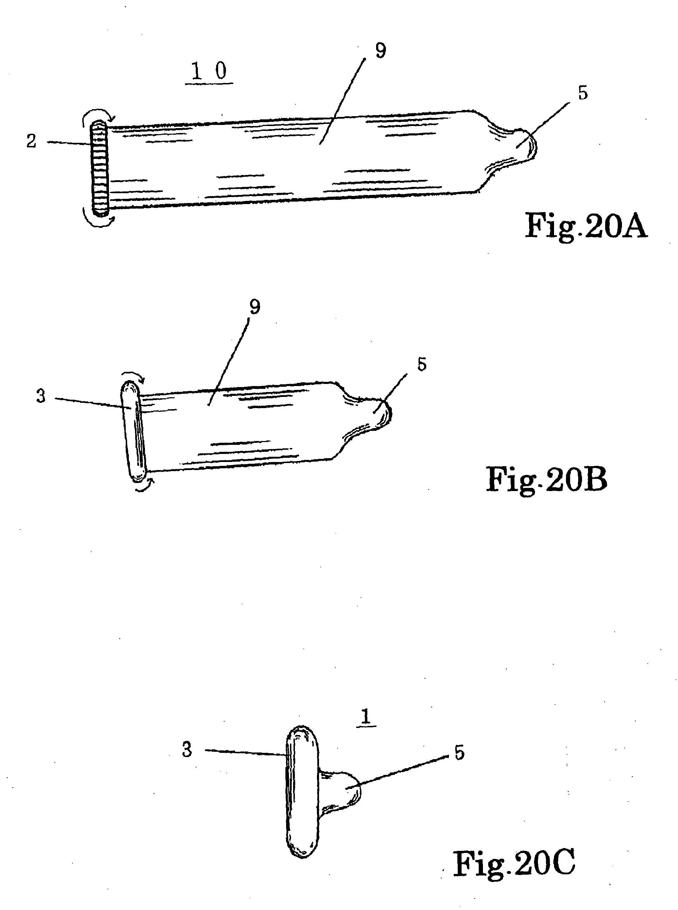

Figs. 20A, 20B and 20C show a method for producing a condom with a wearing auxiliary device. The method for producing a condom with a wearing auxiliary device using a condom and a wearing auxiliary device comprises placing the inner periphery of the wearing auxiliary device adjacent to the outer wall of the neighborhood of the open end of the condom and then rolling up the condom so that the outer wall thereof turns inside using the wearing auxiliary device as a core to form a rolled-up part in the condom.

Here, the production method will be described with reference to the condom shown in Fig. 1 as an example. Fig. 20A shows a condom 10 before a rolled-up part 3 is formed, wherein reference numeral 9 denotes a condom body; reference numeral 5 denotes a teat; and reference numeral 2 denotes a wearing auxiliary device. The wearing auxiliary device 2 is placed such that it is brought into contact with a ring (not shown) formed integrally with the condom body 9 at the open end of the condom (that is, it is attached to the neighborhood of the open end of the condom). Then, the condom is rolled up toward the tip thereof to the closed end so that the outer wall of the condom turns inside (as shown by the arrow) using the ring and the wearing auxiliary device 2 as a core, forming the rolled-up part 3. Fig. 20B shows the step during the formation of the rolled-up part 3, and Fig. 20C shows the condom 1 in the step in which the formation of the rolled-up part 3 is completed. The condom shown in Fig. 20C corresponds to the side view of the one shown in Fig. 1.

As described above, the present invention provides a condom comprising a condom and a wearing auxiliary device, wherein the use of the wearing auxiliary device increases the size or thickness (diameter) of the rolled-up part of the condom. This provides the advantage that the application of the condom during sex acts becomes very easy. In addition, as the wearing auxiliary device is constructed of a coil-shaped member or a polymeric material having rubber elasticity, it is elastic, which provides the advantage that the removal after it is applied can be performed very easily. Since the wearing auxiliary device of the present invention is not exposed to the eye until immediately before the application to the penis is completed, its application can be performed with the feeling similar to conventional condoms. Moreover, the wearing auxiliary device has a simple structure and is easily produced, and it provides the effect that conventional production apparatus can be used as it is for forming the rolled-up part using the auxiliary device as a core; and almost the same amount of packaging materials are used for packaging for commercialization.

## Claims

1. A condom with a wearing auxiliary device comprising a condom made of a natural rubber or a synthetic rubber and a wearing auxiliary device, comprising a structure in which the wearing auxiliary device is used as a core material of the rolled-up part which is formed by rolling-up the condom body from the open end of the condom toward the closed end so that the outer wall of the condom turns inside, wherein the wearing auxiliary device comprises a wearing auxiliary device selected from the group consisting of a wearing auxiliary device (a) comprising a coil-shaped member composed of a material selected from the group consisting of a rubber-like elastic substance, a polymer foam, a polymeric substance and a metal; and a wearing auxiliary device (b) composed of a polymeric material having rubber elasticity.

2. The condom with a wearing auxiliary device according to claim 1, wherein the cross-section of the coil-shaped member of the wearing auxiliary device (a) is generally circular or flat rectangular.

3. The condom with a wearing auxiliary device according to claim 1, wherein the coil-shaped member of the wearing auxiliary device (a) has a structure in which both ends are joined to form a ring or both ends are left as free ends.

4. The condom with a wearing auxiliary device according to claim 1, wherein the polymeric material having rubber elasticity of the wearing auxiliary device (b) is a material selected from the group consisting of polyurethane, polyurethane foam, natural rubber, synthetic rubber and synthetic rubber foam.

5. The condom with a wearing auxiliary device according to claim 1, wherein the wearing auxiliary device (b) has a structure in which a constriction or a notch is provided at any given location in the longitudinal direction thereof.

6. The condom with a wearing auxiliary device according to claim 1, wherein the polymeric material having rubber elasticity of the wearing auxiliary device (b) has a structure which is formed into a ring shape, or in which both ends are joined or left as free ends.
